# EUROPÄISCHE PATENTANMELDUNG

(11) **EP 2 090 917 A2**
(43) Veröffentlichungstag der Anmeldung: **19.08.2009**
(21) Anmeldenummer: 09151906.6
(22) Anmeldetag: 03.02.2009
(51) Int. Cl.: G02B 21/00

(54) **Haltevorrichtung und Mikroskopanordnung**

(30) Priorität: 18.02.2008 DE 102008009678
(71) Anmelder: Carl Zeiss Surgical GmbH, 73447 Oberkochen (DE)
(72) Erfinder: Kolster, Daniel, 73447, Oberkochen (DE)
(74) Vertreter: Müller & Schubert

(57) **Zusammenfassung**

Es wird unter anderem eine Haltevorrichtung (10) zum Kippen und Schwenken einer an der Haltevorrichtung (10) befestigten Einrichtung, insbesondere eines Mikroskops, beschrieben, die eine Befestigungsvorrichtung (11) zum Befestigen der Einrichtung, eine Kippachse (12) zum Kippen der Befestigungsvorrichtung (11) und eine Schwenkachse (13) zum Schwenken der Befestigungsvorrichtung (11) aufweist. Um den erforderlichen Bauraum zu reduzieren und Kabel durch die Achsen führen zu können ist vorgesehen, dass die Kippachse (12) und die Schwenkachse (13) in einer Ebene liegen und derart zueinander ausgerichtet sind, dass sie sich schneiden. Das Kippen und Schwenken kann unter Zuhilfenahme eines besonders ausgestalteten Getriebes (14) realisiert werden. Die Haltevorrichtung (10) weist vorteilhaft zwei unabhängig voneinander betätigbare Antriebe (15: 18) auf. Allein die Antriebsrichtung der Antriebe (15; 18) entscheidet über eine Kippbewegung, eine Schwenkbewegung oder eine kombinierte Kipp-/Schwenkbewegung. Des Weiteren wird eine Mikroskopanordnung beschrieben.

## Beschreibung

Die vorliegende Erfindung betrifft zunächst eine Haltevorrichtung zum Kippen und Schwenken einer an der Haltevorrichtung befestigten Einrichtung, insbesondere eines Mikroskops, gemäß dem Oberbegriff von Patentanspruch 1. Weiterhin betrifft die Erfindung auch eine Mikroskopanordnung.

Haltevorrichtungen, an denen Einrichtungen kippbar und schwenkbar befestigt sind, sind bereits in den vielfältigsten Ausgestaltungen bekannt. Beispielsweise kann es sich bei solchen Einrichtungen um Mikroskope, etwa Operationsmikroskope, und dergleichen handeln. Im weiteren Verlauf wird die Erfindung des Öfteren anhand von Mikroskopen, und hier insbesondere anhand von Operationsmikroskopen, beschrieben, ohne dass die Erfindung jedoch auf diese eine bestimmte Anwendungsform beschränkt wäre.

Operationsmikroskope beispielsweise müssen über dem Operationsfeld frei positionierbar sein. Bisher ist es üblich, dass die dabei durchgeführten Kippbewegungen und Schwenkbewegungen immer in zwei getrennten Achsen durchgeführt werden. Eine derartige Lösung ist beispielsweise in der DE 102 60 888 A1 beschrieben. Eine dort beschriebene Mikroskophalterung umfasst eine Befestigungsvorrichtung zum Befestigen eines Mikroskops, eine Drehachse, die eine Drehung des Mikroskops ermöglicht, und eine Schwenkachse, die ein Verschwenken des Mikroskops ermöglicht. Weiterhin ist auch noch eine Kippachse vorgesehen, um die das Mikroskop gekippt werden kann. Die einzelnen Achsen sind dabei räumlich voneinander getrennt angeordnet. Für eine derartige Ausgestaltung ist, was ein Schwenken und Kippen des Mikroskops betrifft, ein großer Bauraum erforderlich.

Ausgehend vom genannten Stand der Technik liegt der vorliegenden Erfindung die Aufgabe zugrunde, eine Haltevorrichtung der eingangs genannten Art derart weiterzubilden, dass der erforderliche Bauraum reduziert werden kann. Weiterhin soll eine entsprechend verbesserte Mikroskopanordnung bereitgestellt werden.

Diese Aufgabe wird erfindungsgemäß gelöst durch die Haltevorrichtung mit den Merkmalen gemäß dem unabhängigen Patentanspruch 1 sowie die Mikroskopanordnung mit den Merkmalen gemäß dem unabhängigen Patentanspruch 15. Weitere Merkmale und Details der Erfindung ergeben sich aus den Unteransprüchen, der Beschreibung sowie den Zeichnungen. Merkmale und Details, die im Zusammenhang mit der erfindungsgemäßen Haltevorrichtung beschrieben sind, geltend dabei selbstverständlich auch im Zusammenhang mit der erfindungsgemäßen Mikroskopanordnung, und umgekehrt.

Gemäß dem ersten Aspekt der Erfindung wird eine Haltevorrichtung zum Kippen und Schwenken einer an der Haltevorrichtung befestigten Einrichtung, insbesondere eines Mikroskops, bereitgestellt, aufweisend eine Befestigungsvorrichtung zum Befestigen der Einrichtung, eine Kippachse zum Kippen der Befestigungsvorrichtung und eine Schwenkachse zum Schwenken der Befestigungsvorrichtung. Die Haltevorrichtung ist erfindungsgemäß dadurch gekennzeichnet, dass die Kippachse und die Schwenkachse in einer Ebene liegen und derart zueinander ausgerichtet sind, dass sie sich schneiden.

Durch die erfindungsgemäße Haltevorrichtung wird ein kompakter Kipp- und Schwenkmechanismus realisiert, der insbesondere im Zusammenhang mit Mikroskopen, beispielsweise Operationsmikroskopen, eingesetzt werden kann. Im Gegensatz dazu haben die bisher bekannten Haltevorrichtungen immer einen größeren Bauraum benötigt, da die Achsen räumlich voneinander getrennt lagen. Für den Betrieb erforderliche Elemente, wie beispielsweise Kabel und dergleichen, mussten bei den bekannten Lösungen stets durch die zwei Achsen, oder an diesen vorbei gelegt werden. Durch die erfindungsgemäße Ausgestaltung der Haltevorrichtung kann nunmehr der erforderliche Bauraum erheblich reduziert werden. Weiterhin können die Kabel einfach und bequem durch die Achsen hindurchgeführt werden.

Die erfindungsgemäße Haltevorrichtung verfügt zunächst über eine Befestigungsvorrichtung, an der die zu kippende und zu verschwenkende Einrichtung befestigt ist. Im Zusammenhang mit einem Mikroskop kann es sich bei der Befestigungseinrichtung beispielsweise um den Mikroskophalter handeln. Weiterhin vorgesehen ist eine Kippachse, um die die Befestigungseinrichtung gekippt werden kann, sowie eine Schwenkachse, um die die Befestigungseinrichtung geschwenkt werden kann.

Damit der erforderliche Bauraum reduziert werden kann, ist nunmehr vorgesehen, dass beide Achsen in einer einzigen Ebene liegen. Weiterhin ist vorgesehen, dass sich die beiden Achsen schneiden. Vorteilhaft kann dabei vorgesehen sein, dass die beiden Achsen in einer Ebene senkrecht zueinander ausgerichtet sind. Durch diese Ausgestaltung kann zusätzlich auch die Antriebseinrichtung, die für den Kippvorgang und den Schwenkvorgang, stark vereinfacht werden, was einen weiteren Vorteil der erfindungsgemäßen Haltevorrichtung bedeutet. Nicht ausschließliche Beispiele, wie dies im Einzelnen realisiert werden kann, werden im weiteren Verlauf der Beschreibung näher erläutert.

Vorteilhaft kann zur Ausführung einer Kippbewegung der Befestigungsvorrichtung um die Kippachse und zur Ausführung einer Schwenkbewegung der Befestigungsvorrichtung um die Schwenkachse ein einziges Getriebe vorgesehen sein. Dabei ist die Erfindung nicht auf bestimmte Ausführungsformen für das Getriebe beschränkt. Generell kann es sich bei dem Getriebe um jegliche mechanische Vorrichtung zum Übertragen und Wandeln von Drehbewegungen, Drehrichtungen und Drehmomenten handeln. Beispielsweise kann es sich bei dem einzusetzenden Getriebe um eine Art angetriebenes Kardangelenk in Form eines invertierten Differentialgetriebes oder dergleichen handeln.

Vorteilhaft kann die Haltevorrichtung zwei unabhängig von einander betätigbare Antriebe aufweisen, mittels derer eine Kippung der Befestigungsvorrichtung um die Kippachse und eine Schwenkung der Befestigungsvorrichtung um die Schwenkachse durchführbar ist. Somit führen zwei Antriebe die Kippbewegung, Schwenkbewegung oder eine kombinierte Bewegung der an der Befestigungsvorrichtung befestigten Einrichtung aus.

Vorteilhaft ist vorgesehen, dass die beiden Antriebe gleichläufig und/oder gegenläufig betätigbar ausgebildet sind.

Auf diese Weise kann eine Situation realisiert werden, in der nur ein einziger Antrieb in einer bestimmten Antriebsrichtung betrieben wird, während der andere Antrieb nicht betätigt ist. Es ist ebenso möglich, dass beide Antriebe gleichzeitig betätigt werden. Dann kann beispielsweise eine Situation realisiert werden, in der beide Antriebe gleichläufig, das heißt mit gleicher Antriebsrichtung, betätigt werden. Ebenso ist eine Situation realisierbar, in der beide Antriebe gegenläufig, das heißt in entgegengesetzter Antriebsrichtung, betätigt werden.

Beispielsweise kann vorgesehen sein, dass die beiden Antriebe mit unterschiedlichen Drehrichtungen betrieben werden können. Dabei kann jeder der Antriebe jeweils für sich allein betrachtet unterschiedliche Drehrichtungen aufweisen, das heißt, mit unterschiedlichen Drehrichtungen betrieben werden. Ebenso ist denkbar, dass die beiden Antriebe im Vergleich zueinander jeweils eine gleiche und/oder unterschiedliche Drehrichtung aufweisen. Drehen beide Antriebe gleichläufig, das heißt in die gleiche Richtung, so kann beispielsweise eine Kippbewegung der Einrichtung, etwa des Mikroskops, ausgeführt werden. Drehen beide Antriebe nicht gleichläufig, wobei beispielsweise der eine Antrieb - vorzugsweise in gleichem Maß - entgegengesetzt wie der andere Antrieb dreht, so führt die Einrichtung eine Schwenkbewegung aus. Dreht nur einer der Antriebe, so führt die Einrichtung eine kombinierte Kipp- und Schwenkbewegung aus.

Allein die Antriebsrichtung der Antriebe, beispielsweise deren Drehrichtung, entscheidet dann über eine Kippbewegung, eine Schwenkbewegung oder eine kombinierte Bewegung in Form einer Kipp-/Schwenkbewegung.

Vorzugsweise weist die Haltevorrichtung einen Lagerbock auf, durch den sich die Kippachse und die Schwenkachse erstrecken. Dieser Lagerbock kann beispielsweise im Innern des Getriebes angeordnet sein. Dann ist es beispielsweise möglich, Kabel und dergleichen durch die Mitte zu führen. Der Lagerbock dient zur Abstützung der erforderlichen Lager, bei denen es sich beispielsweise um Axiallager, um Radiallager, um kombinierte Axial/Radial-Lager, oder dergleichen handeln kann.

Die vorliegende Erfindung ist nicht auf bestimmte Ausführungsformen beschränkt, wie die Kippachse und die Schwenkachse ausgebildet zu sein haben. Vorteilhaft kann vorgesehen sein, dass die Kippachse und die Schwenkachse als mit dem Lagerbock verbundene Zapfen ausgebildet sind. Dabei können die Zapfen je nach Bedarf lösbar oder nicht lösbar mit dem Lagerbock verbunden sein. In einer bevorzugten Ausführungsform kann beispielsweise vorgesehen sein, dass die Zapfen jeweils als einschraubbare Zapfen ausgeführt sind, die in den Lagerbock eingeschraubt und mit diesem auf diese Weise fest verschraubt werden.

Um die Drehbewegung auf das Getriebe übertragen zu können, weist jeder Antrieb in einer bevorzugten Ausgestaltung eine Antriebsschnecke auf. Die Antriebsschnecke wiederum kann über einen geeigneten Motor angetrieben werden.

Das Getriebe kann vorteilhaft zwei Schneckenräder aufweisen, die entlang der Kippachse angeordnet sind. Jeweils ein Antrieb treibt dann ein Schneckenrad an. Beispielsweise kann vorgesehen sein, dass ein erster Antrieb, bestehend aus erstem Motor und erster Antriebsschnecke, ein erstes Schneckenrad antreibt, während ein zweiter Antrieb, bestehend aus zweitem Motor und zweiter Antriebsschnecke, das zweite Schneckenrad antreibt. Somit kann jedes der Schneckenräder individuell und unabhängig vom jeweils anderen Schneckenrad angetrieben werden. Vorteilhaft sind die Schneckenräder derart entlang der Kippachse angeordnet, dass sich die übrigen Elemente des Getriebes zwischen den beiden Schneckenrädern befinden. Dies gilt dann insbesondere auch für den Lagerbock und die Befestigungsvorrichtung für die Einrichtung, etwa das Mikroskop.

In weiterer Ausgestaltung kann jedes der Schneckenräder mit einem Antriebskegelrad verbunden sein. Beispielsweise kann vorgesehen sein, dass jeweils ein Schneckenrad und ein Antriebskegelrad als ein einziges Bauteil ausgebildet sind. Wenn das Schneckenrad und das Antriebskegelrad fest miteinander verbunden werden, können diese auch als zwei Einzelbauteile vorliegen.

Insbesondere kann vorgesehen sein, dass die Antriebskegelräder im Lagerbock gelagert sind.

Vorzugsweise kann vorgesehen sein, dass das Getriebe ein Abtriebskegelrad aufweist, das fest mit der Befestigungsvorrichtung verbunden ist. Dabei kann insbesondere vorgesehen sein, dass das Abtriebskegelrad entlang der Schwenkachse angeordnet ist. Das Abtriebskegelrad ist insbesondere ortsfest mit der Befestigungsvorrichtung, beispielsweise einem Mikroskophalter, verbunden. Vorteilhaft ist das Abtriebskegelrad derart ausgebildet und angeordnet, dass es mit den weiter oben beschriebenen Antriebskegelrädern kämmen kann.

In weiterer Ausgestaltung kann das Getriebe ein Gegenkegelrad aufweisen, welches am Lagerbock gelagert ist. Auch dieses Gegenkegelrad kann vorteilhaft entlang der Schwenkachse angeordnet sein. Dies ist insbesondere dann vorteilhaft, wenn das weiter oben beschriebene Abtriebskegelrad und das Gegenkegelrad gegenläufig sind. Das Gegenkegelrad ist lediglich optional und kann auch weggelassen oder durch eine gelagerte Achse ersetzt werden.

Vorzugsweise kann die Haltevorrichtung eine Einrichtung zur Lagebestimmung aufweisen. Mittels dieser Einrichtung zur Lagebestimmung kann dann die Lage einer an der Haltevorrichtung befestigten Einrichtung, beispielsweise eines Mikroskops, insbesondere eines Operationsmikroskops, bestimmt werden.

Um die Lage der an der Haltevorrichtung befestigten Einrichtung bestimmen zu können, gibt es verschiedene Möglichkeiten, so dass die Erfindung nicht auf bestimmte Ausgestaltungen der Einrichtung zur Lagebestimmung beschränkt ist.

Einige vorteilhafte, jedoch nicht ausschließliche Beispiel hierzu werden nachfolgend beschrieben.

Beispielsweise kann die Einrichtung zur Lagebestimmung als Encodereinrichtung, vorteilhaft als Magnetencodereinrichtung ausgebildet sein. Mittels einer solchen Encodereinrichtung ist es beispielsweise möglich, die Stellung einzelner Räder, beispielsweise einzelner wie oben beschriebener Schneckenräder, zueinander zu bestimmen.

Eine Möglichkeit ist deshalb die Detektierung der Stellung der Schneckenräder zueinander, beispielsweise mittels einer Magnetencodereinrichtung. Hierbei wird je ein Magnet, der sich über je einem Sensorelement (beispielsweise einem Sensor-IC) befindet, fest mit dem jeweiligem Schneckenrad verbunden. Durch Verrechnung der beiden Encoderwerte, lässt sich die Position der an der Haltevorrichtung befestigten Einrichtung bestimmen. Weitere Möglichkeiten für Einrichtungen zur Lagebestimmung sind etwa Potentiometer, die vorzugsweise je in einer Antriebseinheit / Getriebeeinheit integriert werden. Ist eine Referenzfahrt erlaubt, kann auch ein Inkrementalgeber (nicht Absolut) an den Antrieben für die Positionsbestimmung verwendet werden.

Die Lage der an der Haltevorrichtung befestigten Einrichtung, etwa eines Mikroskops, insbesondere eines Operationsmikroskops, kann aber auch auf andere Weise erfolgen, beispielsweise direkt mit zwei im rechten Winkel angeordneten Winkelsensoren, wie sie beispielsweise auch in elektronischen Wasserwaagen eingesetzt werden. In einem solchen Fall erfolgt die Lagebestimmung allerdings direkt in der Einrichtung, so dass in einem solchen Fall die Einrichtung zur Lagebestimmung nicht Bestandteil der Haltevorrichtung sein muss, sondern auch Bestandteil der entsprechenden Einrichtung, etwa des Mikroskops, insbesondere des Operationsmikroskops, sein kann.

Vorteilhaft kann die Haltevorrichtung eine Einrichtung zur Befestigung an einem Stativ aufweisen. Das Stativ dient zur Aufnahme, insbesondere zur beweglichen Aufnahme, der entsprechenden Einrichtung, etwa des Mikroskops, insbesondere des Operationsmikroskops. Je nach Ausgestaltung kann es sich bei der Haltevorrichtung allerdings auch um das Stativ selbst, oder aber einen Bestandteil eines solchen Stativs, handeln.

Durch eine geeignete Auswahl und Kombinatorik des Getriebes sowie der einzelnen Getriebeglieder kann dieses vorzugsweise als selbsthemmendes oder nicht selbsthemmendes System ausgebildet werden. Selbsthemmende Getriebe zeichnen sich grundsätzlich dadurch aus, dass sie eine Abtriebswelle in einer angefahrenen Position festhalten können, und dass die Abtriebswelle nur durch ein Drehen der Antriebswelle verdreht werden kann. Bei einem nicht selbsthemmenden System könnte die an der Befestigungsvorrichtung befestigte Einrichtung, etwa ein Mikroskop, beispielsweise mit einer Feder oder Federeinrichtung, oder nach dem Prinzip der elektronischen Feder, und/oder mit Hilfe von elektronischen Bremsen ausbalanciert und von Hand frei positioniert werden. Für ein selbsthemmendes System können vorteilhaft Scheckengetriebe verwendet werden. Es ist aber auch denkbar, dass anstelle von Schneckenrädern auch stirnverzahnte Räder verwendet werden können, sofern das auftretende Moment kompensiert werden kann, beispielsweise durch Motorbremsen oder dergleichen. Diese Ausgestaltung ist beispielsweise dann vorteilhaft, wenn man die Bremsen auf Knopfdruck oder Befehl hin öffnen möchte, um die Einrichtung, beispielsweise das Mikroskop, von Hand zu positionieren.

Die erfindungsgemäße Haltevorrichtung bietet ferner die Möglichkeit, Kabel und dergleichen auf einmal durch die zwei Achsen zu führen. Dabei werden die Kabel, ohne sich zu verlängern oder zu verkürzen, vorteilhaft entlang der neutralen Faser gebogen.

Ein wesentlicher Vorteil der erfindungsgemäßen Haltevorrichtung mit dem besonderen Antrieb und dem besonderen Getriebe besteht darin, dass die doppelten Komponenten verwendet werden. Dadurch kann nicht nur Geld eingespart werden, sondern die einzelnen Komponenten können auch von der Festigkeit her kleiner ausgelegt werden. Das Moment, welches auf die Kippachse wirkt, teilt sich beispielsweise auf die beiden Schneckenräder auf. Somit trägt nicht nur ein Schneckenrad das Moment, sondern zwei. Dies hat enorme Auswirkungen auf die Baugröße. Wird ein Gegenkegelrad verwendet, so sind immer mehrere Zähne im Eingriff, um das Moment aufzunehmen, statt nur einem, wenn der Kippvorgang betrachtet wird. Dies lässt in der Auslegung ein kleineres Zahnmodul zu. Allein die Drehrichtung der beiden Antriebe bestimmt die Kipp- oder Schwenkbewegung.

In einer vorteilhaften Ausführungsform kann die Haltevorrichtung zwei angetriebene Antriebskegelräder aufweisen, sowie ein Abriebskegelrad, das in die beiden angetriebenen Antriebskegelräder eingreift. Die Kegelräder sind vorteilhaft in einem Lagerbock in einer Ebene gelagert, und zwar derart, dass sich die Achsen schneiden.

Gemäß einem zweiten Aspekt der Erfindung wird eine Mikroskopanordnung bereitgestellt, die zunächst über eine wie vorstehend beschriebene erfindungsgemäße Haltevorrichtung verfügt. Bezüglich der Haltevorrichtung wird deshalb auch auf die vorstehenden Ausführungen vollinhaltlich Bezug genommen und hiermit verwiesen. Weiterhin verfügt die Mikroskopanordnung über ein Mikroskop das an der Haltevorrichtung befestigt ist. Vorteilhaft kann weiterhin auch noch ein Stativ vorgesehen sein, an dem die Haltevorrichtung befestigt ist.

Bei dem Mikroskop kann es sich besonders vorteilhaft um ein Operationsmikroskop handeln, das in verschiedenen medizinischen Bereichen eingesetzt werden kann. Beispielsweise kann es sich bei dem Operationsmikroskop um ein ophtalmologisches Mikroskop handeln.

Die vorliegende Erfindung betrifft vorteilhaft solche Ausgestaltungen, bei denen die Antriebe zur Bewegung der Einrichtung, etwa eines Mikroskops, insbesondere eines Operationsmikroskops, in der Haltevorrichtung vorgesehen sind. Es sind jedoch auch solche Ausführungsformen möglich, bei denen die Antriebe in der mit der Haltevorrichtung verbundenen Einrichtung selbst vorgesehen sind.

Die Erfindung wird nun anhand eines Ausführungsbeispiels unter Bezugnahme auf die beiliegenden Zeichnungen näher erläutert. Es zeigen
- Figur 1: in perspektivischer Ansicht eine erfindungsgemäße Haltevorrichtung;
- Figur 2: in schematischer Schnittdarstellung die Draufsicht auf ein erstes Ausführungsbeispiel eines Getriebes, welches in der Haltevorrichtung verwirklicht ist; und
- Figur 3: in schematischer Schnittdarstellung die Draufsicht auf ein zweites Ausführungsbeispiel eines Getriebes, welches in der Haltevorrichtung verwirklicht ist.

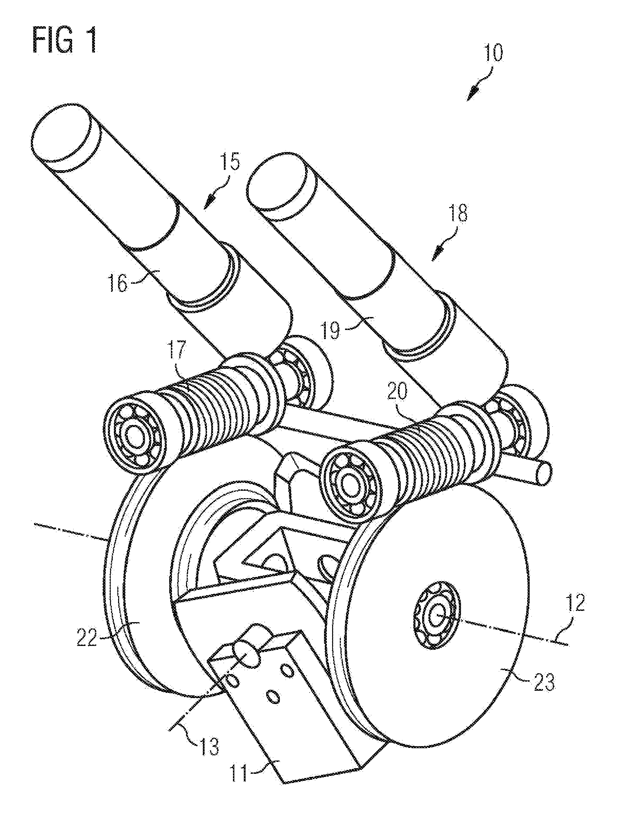

In Figur 1 ist eine Haltevorrichtung 10 dargestellt, die über eine Befestigungsvorrichtung 11 für eine Einrichtung (nicht dargestellt) verfügt. Im vorliegenden Beispiel soll die Befestigungsvorrichtung 11 als Halter für ein Mikroskop, beispielsweise ein Operationsmikroskop, ausgebildet sein.

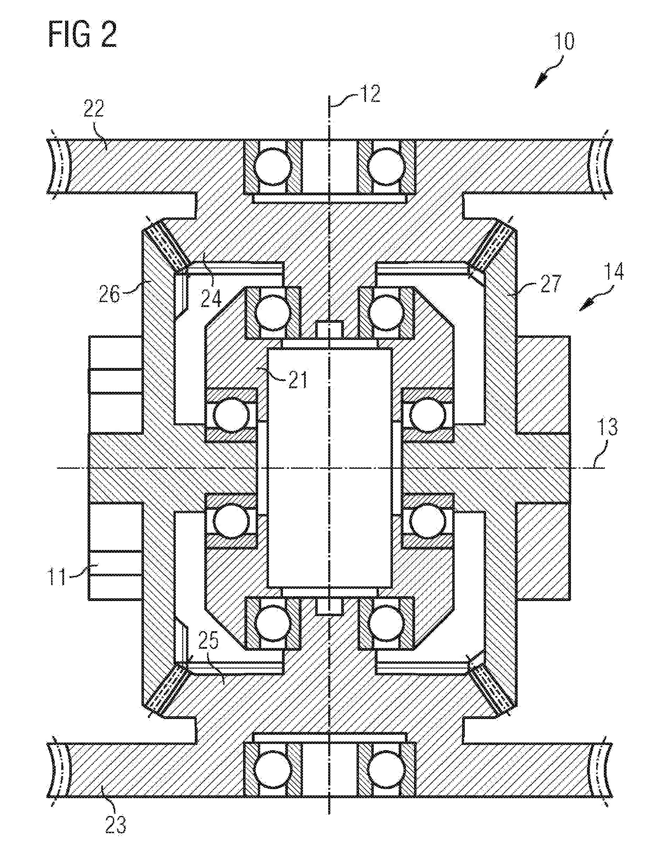

Die Befestigungsvorrichtung 11 kann über eine Kippachse 12 gekippt, und über eine Schwenkachse 13 geschwenkt werden. Dies geschieht im Wesentlichen über zwei Schneckenräder 22, 23, die entlang der Kippachse 12 angeordnet sind. Die Schneckenräder 22, 23 stellen Bestandteile eines Getriebes dar, das im Zusammenhang mit der Figur 2 näher erläutert wird.

Jedes der Schneckenräder 22, 23 wirkt mit einem eigenen Antrieb zusammen. Ein erster Antrieb 15 verfügt über einen Motor 16 und eine über den Motor 16 angetriebene Antriebsschnecke 17. Die Antriebsschnecke 17 wirkt mit dem Schneckenrad 22 zusammen. Ein zweiter Antrieb 18 verfügt über einen Motor 19 und eine über den Motor 19 angetriebene Antriebsschnecke 20. Die Antriebsschnecke 20 wirkt mit dem Schneckenrad 23 zusammen.

Durch die beiden unabhängig von einander betätigbaren Antriebe 15, 18 ist eine Kippung der Befestigungsvorrichtung 11 um die Kippachse 12, und eine Schwenkung der Befestigungsvorrichtung 11 um die Schwenkachse 13 durchführbar. Somit führen zwei Antriebe 15, 18 die Kippbewegung, Schwenkbewegung oder die kombinierte Bewegung der an der Befestigungsvorrichtung 11 befestigten Einrichtung aus. Drehen beide Antriebe 15, 18 gleichläufig, das heißt in die gleiche Richtung, so kann beispielsweise eine Kippung der Einrichtung, etwa des Mikroskops, ausgeführt werden. Drehen beide Antriebe 15, 18 nicht gleichläufig, wobei beispielsweise der eine Antrieb in gleichem Maß entgegengesetzt wie der andere Antrieb dreht, so führt die Einrichtung eine Schwenkbewegung aus. Dreht nur einer der Antriebe 15, 18, so führt die Einrichtung eine kombinierte Kipp- und Schwenkbewegung aus.

In Figur 2 ist das Getriebe 14 der Haltevorrichtung 10 in größerem Detail dargestellt. Zunächst ist in Figur 2 zu erkennen, dass die Kippachse 12 und die Schwenkachse 13 in einer Ebene liegen, und dass beide Achsen derart ausgerichtet sich, dass sie sich - insbesondere orthogonal - schneiden. Das Getriebe 14 kann über eine geeignete Anbindung an einem Stativ (nicht dargestellt) oder dergleichen befestigt werden. Das Mikroskop (nicht dargestellt) an sich ist an der Befestigungsvorrichtung 11 befestigt. Wie im Zusammenhang mit Figur 1 bereits erläutert wurde, treibt der Antrieb 15 das Schneckenrad 22 an, während das Schneckenrad 23 durch den Antrieb 18 angetrieben wird. Beide Schneckenräder 22, 23 sind entlang der Kippachse 12 angeordnet. In Figur 2 sind weiterhin Antriebskegelräder 24, 25 dargestellt, wobei das Schneckenrad 22 mit dem Antriebskegelrad 24, und das Schneckenrad 23 mit dem Antriebskegelrad 25 jeweils als ein Teil ausgebildet ist.

Die Antriebskegelräder 24, 25 sind in einem Lagerbock 21 gelagert. Dieser Lagerbock 21 ist im Beispiel im Innern des Getriebes 14 angeordnet. Dadurch wird es möglich. Kabel und dergleichen durch die Mitte des Getriebes 14 zu führen. Allerdings sind auch andere Positionen möglich, beispielsweise, dass alle Kegelräder innerhalb des Lagerbocks 21 liegen und die Befestigungsvorrichtung 11 im Zentrum liegt. Wichtig ist lediglich, dass sich die Kippachse 12 und die Schwenkachse 13 schneiden, also in einer Ebene liegen.

Weiterhin ist ein Abtriebskegelrad 26 vorgesehen, welches im Beispiel ortsfest mit der Befestigungsvorrichtung 11 verbunden ist. Darüber hinaus ist auch noch ein Gegenkegelrad 27 vorgesehen, welches am Lagerbock 21 gelagert ist, da Abtriebskegelrad 26 und Gegenkegelrad 27 gegenläufig sind. Das Abtriebskegelrad 26 und das Gegenkegelrad 27 sind entlang der Schwenkachse 13 angeordnet. Das Gegenkegelrad 27 ist nicht zwingend erforderlich. Es könnte beispielsweise auch durch eine gelagerte Achse ersetzt werden.

Ein wesentlicher Vorteil der erfindungsgemäßen Haltevorrichtung 10 mit dem besonderen Antrieb und dem besonderen Getriebe 14 besteht darin, dass die doppelten Komponenten verwendet werden. Das Moment, welches auf die Kippachse 12 wirkt, teilt sich beispielsweise auf die beiden Schneckenräder 22, 23 auf. Somit trägt nicht nur ein Schneckenrad das Moment, sondern zwei. Wird ein Gegenkegelrad 27 verwendet, so sind immer mehrere Zähne, beispielsweise vier, im Eingriff, um das Moment aufzunehmen, statt nur einem, wenn der reine Kippvorgang betrachtet wird. Allein die Drehrichtung der beiden Antriebe 15, 18 (Figur 1) bestimmt die Kipp- oder Schwenkbewegung.

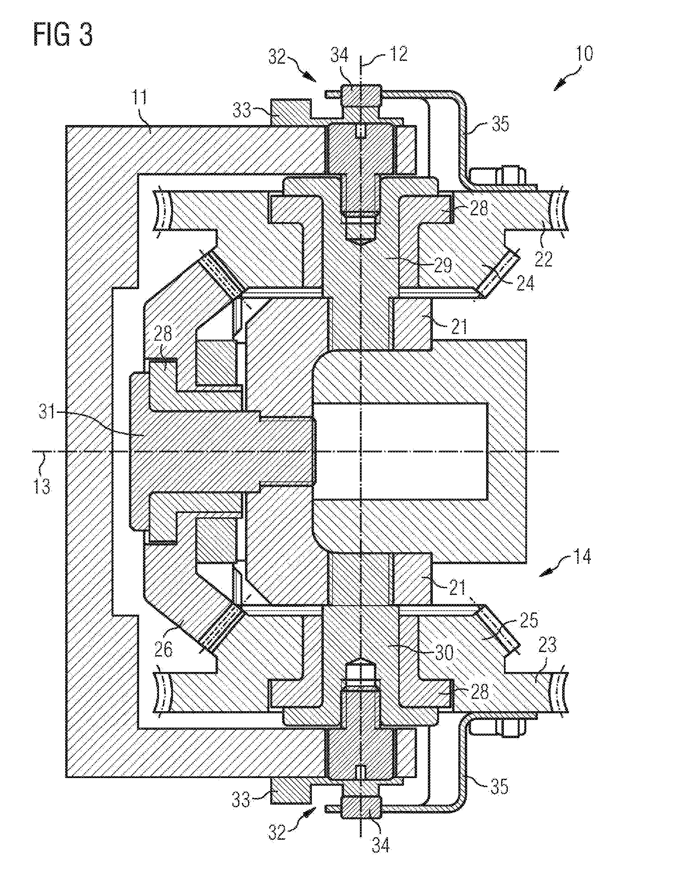

In Figur 3 ist eine weitere Ausführungsform einer Haltevorrichtung 10 beschireben, die eine Befestigungsvorrichtung 11 für ein Mikroskop aufweist und die über ein entsprechendes Getriebe 14 verfügt. Der Grundaufbau der Haltevorrichtung 10 entspricht dabei der in Figur 2 dargestellten Haltevorrichtung 10, so dass zunächst auf die entsprechenden Ausführungen vollinhaltlich Bezug genommen und verwiesen wird. Weiterhin sind baugleiche Elemente mit identischen Bezugsziffern versehen.

Im Vergleich zu dem in Figur 2 gezeigten Beispiel ist in Figur 3 eine Variante ohne ein Gegenkegelrad mit einem offenen Lagerbock 21 dargstellt. Die Lager 28 sind als kombinierte Axial/Radiallager ausgebildet. Die Kippachse 12 und die Schwenkachse 13 sind bei diesem Ausführungsbeispiel als einschraubbare Zapfen 29, 30, 31 ausgeführt, die fest mit dem Lagerbock 21 verschraubt werden beziehungsweise sind.

Zusätzlich verfügt die Haltevorrichtung 10 der Figur 3 über eine Einrichtung 32 zur Lagebestimmung. Über die Einrichtung 32 zur Lagebestimmung kann die Lage des an der Haltevorrichtung 10 angeordneten Mikroskops bestimmt werden. Dazu gibt es verschiedene Möglichkeiten. In Figur 3 ist eine Variante dargestellt, bei der die Einrichtung 32 zur Lagebestimmung als Encodereinrichtung, insbesondere als Magnetencodereinrichtung, ausgebildet ist.

Mittels der Magnetencodereinrichtung 32 kann die Stellung der Schneckenräder 22, 23 zueinander detektiert werden. Hierbei wird je ein Magnet 34, der sich über je einem Sensorelement 33, beispielsweise in Form eines Sensor-ICs, befindet, über einen Magnethalter 35 fest mit dem jeweiligem Schneckenrad 22, 23 verbunden. Durch Verrechnung der beiden Encoderwerte lässt sich die Mikroskopposition bestimmen.

### Bezugszeichenliste

- 10: Haltevorrichtung
- 11: Befestigungsvorrichtung (für ein Mikroskop)
- 12: Kippachse
- 13: Schwenkachse
- 14: Getriebe
- 15: Antrieb
- 16: Motor
- 17: Antriebsschnecke
- 18: Antrieb
- 19: Motor
- 20: Antriebsschnecke
- 21: Lagerbock
- 22: Schneckenrad
- 23: Schneckenrad
- 24: Antriebskegelrad
- 25: Antriebskegelrad
- 26: Abtriebskegelrad
- 27: Gegenkegelrad
- 28: Lager (Axial/Radial-Kombilager)
- 29: Zapfen
- 30: Zapfen
- 31: Zapfen
- 32: Einrichtung zur Lagebestimmung (Magnetencodereinrichtung)
- 33: Sensorelement
- 34: Magnet
- 35: Magnethalter

## Patentansprüche

1. Haltevorrichtung (10) zum Kippen und Schwenken einer an der Haltevorrichtung (10) befestigten Einrichtung, insbesondere eines Mikroskops, aufweisend eine Befestigungsvorrichtung (11) zum Befestigen der Einrichtung, eine Kippachse (12) zum Kippen der Befestigungsvorrichtung (11) und eine Schwenkachse (13) zum Schwenken der Befestigungsvorrichtung (11), **dadurch gekennzeichnet, dass** die Kippachse (12) und die Schwenkachse (13) in einer Ebene liegen und derart zueinander ausgerichtet sind, dass sie sich schneiden.

2. Haltevorrichtung nach Anspruch 1, **dadurch gekennzeichnet, dass** zur Ausführung einer Kippbewegung der Befestigungsvorrichtung (11) um die Kippachse (12) und zur Ausführung einer Schwenkbewegung der Befestigungsvorrichtung (11) um die Schwenkachse (13) ein einziges Getriebe (14) vorgesehen ist.

3. Haltevorrichtung nach Anspruch 1 oder 2, **dadurch gekennzeichnet, dass** diese zwei unabhängig von einander betätigbare Antriebe (15; 18) aufweist, mittels derer eine Kippung der Befestigungsvorrichtung (11) um die Kippachse (12) und eine Schwenkung der Befestigungsvorrichtung (11) um die Schwenkachse (13) durchführbar ist.

4. Haltevorrichtung nach Anspruch 3, **dadurch gekennzeichnet, dass** die Antriebe (15; 18) gleichläufig und/oder gegenläufig betätigbar ausgebildet sind.

5. Haltevorrichtung nach einem der Ansprüche 1 bis 4, **dadurch gekennzeichnet, dass** diese einen Lagerbock (21) aufweist, durch den sich die Kippachse (12) und die Schwenkachse (13) erstrecken.

6. Haltevorrichtung nach Anspruch 5, **dadurch gekennzeichnet, dass** die Kippachse (12) und die Schwenkachse (13) als mit dem Lagerbock (21) verbundene Zapfen (29, 30, 31) ausgebildet sind.

7. Haltevorrichtung nach einem der Ansprüche 3 bis 6, **dadurch gekennzeichnet, dass** jeder Antrieb (15; 18) eine Antriebsschnecke (17; 20) aufweist.

8. Haltevorrichtung nach einem der Ansprüche 2 bis 7, **dadurch gekennzeichnet, dass** das Getriebe (14) zwei Schneckenräder (22, 23) aufweist, die entlang der Kippachse (12) angeordnet sind.

9. Haltevorrichtung nach Anspruch 8, **dadurch gekennzeichnet, dass** jedes der Schneckenräder (22, 23) mit einem Antriebskegelrad (24, 25) verbunden ist.

10. Haltevorrichtung nach Anspruch 9, soweit auf einen der Ansprüche 5 bis 8 rückbezogen, **dadurch gekennzeichnet, dass** die Antriebskegelräder (24, 25) im Lagerbock (21) gelagert sind.

11. Haltevorrichtung nach einem der Ansprüche 2 bis 10, **dadurch gekennzeichnet, dass** das Getriebe (14) ein Abtriebskegelrad (26) aufweist, das fest mit der Befestigungsvorrichtung (11) verbunden ist.

12. Haltevorrichtung nach Anspruch 11, soweit auf einen der Ansprüche 9 oder 10 rückbezogen, **dadurch gekennzeichnet, dass** das Abtriebskegelrad (26) mit den Antriebskegelrädern (24, 25) kämmt.

13. Haltevorrichtung nach einem der Ansprüche 5 bis 12, **dadurch gekennzeichnet, dass** das Getriebe (14) ein Gegenkegelrad (27) aufweist, welches an dem Lagerbock (21) gelagert ist.

14. Haltevorrichtung nach einem der Ansprüche 1 bis 13, **dadurch gekennzeichnet, dass** diese eine Einrichtung (32) zur Lagebestimmung einer an der Haltevorrichtung (10) befestigten Einrichtung aufweist.

15. Mikroskopanordnung, insbesondere Operationsmikroskopanordnung, mit einer Haltevorrichtung (10) nach einem der Ansprüche 1 bis 14, und mit einem Mikroskop, das an der Haltevorrichtung (10) befestigt ist.
